# EUROPÄISCHE PATENTSCHRIFT

(11) **EP 0 614 349 B1**
(45) Veröffentlichungstag und Bekanntmachung des Hinweises auf die Patenterteilung: **28.02.1996**
(21) Anmeldenummer: 92923525.7
(22) Anmeldetag: 16.11.1992
(51) Int. Cl.: A61K 7/06

(54) **VERWENDUNG VON QUATERNIERTEN FETTSÄURETRIALKANOLAMIN-ESTER- SALZEN SOWIE FETTALKOHOLEN UND/ODER FETTALKOHOLPOLYGLYCOL- ETHERN ZUR HERSTELLUNG VON HAARPFLEGEMITTELN**
USE OF QUATERNIZED FATTY ACID TRIALKANOLAMINE ESTER SALTS AND FATTY ALCOHOLS AND/OR FATTY ALCOHOL POLYGLYCOLETHERS FOR THE PRODUCTION OF HAIR CARE COMPOSITIONS
UTILISATION DE SELS D'ESTERS DE TRIALKANOLAMINES D'ACIDES GRAS QUATERNISES ET D'ALCOOLS GRAS ET/OU D'ESTERS POLYGLYCOLIQUES D'ALCOOLS GRAS POUR LA PRODUCTION DE COMPOSITIONS POUR LES SOINS DES CHEVEUX

(30) Priorität: 25.11.1991 DE 4138630
(43) Veröffentlichungstag der Anmeldung: 14.09.1994
(73) Patentinhaber: Henkel Kommanditgesellschaft auf Aktien, D-40191 Düsseldorf (DE)
(72) Erfinder: HENSEN, Hermann, D-5657 Haan (DE); STUHRMANN, Dagmar, Miami, FL 33102 (US); PONSATI OBIOLS, Oriol, E-08025 Barcelona (ES); PRAT QUERALT, Esther, E-08328 Alella (ES)
(86) Internationale Anmeldenummer: EP9202633
(87) Internationale Veröffentlichungsnummer: WO9310748

(56) Entgegenhaltungen:
- EP-A- 0 252 441
- EP-A- 0 284 036
- EP-A- 0 299 787
- EP-A- 0 309 052
- EP-A- 0 367 939

## Beschreibung

### Gebiet der Erfindung

Die Erfindung betrifft die Verwendung von Gemischen, enthhaltend quaternierte Fettsäuretrialkanolaminester-Salze sowie Fettalkohole und/oder Fettalkoholpolyglycolether zur Herstellung von Haarpflegemitteln.

### Stand der Technik

Schädigungen der Haarstruktur sind die Folge häufigen Bleichens, Dauerwellens, Färbens, starker UV-Belastung, Waschens der Haare mit entfettenden Tensiden sowie das Ergebnis einer normalen Alterung. Das Haar wird spröde und verliert seinen Glanz. Des weiteren findet beim Kämmen des Haares eine elektrostatische Aufladung statt, während die aufgerauhte Haaroberfläche Anlaß zu Verfilzungen und Verknotungen des Haares gibt und auf diese Weise das Kämmen erschwert. Haarpflegemittel mit einer kämmbarkeitsverbessernden Wirkung haben daher erhebliche Bedeutung auf dem Kosmetikmarkt erlangt. Derartige Mittel können beispielsweise in Form einer Spülung, eines Aerosol-Schaums oder auch in Form von Emulsionen (Creme-Rinses) nach der Haarwäsche im noch nassen Haar verteilt und entweder nach einigen Minuten Einwirkungszeit ausgespült oder auf dem Haar belassen werden.

Als Wirkstoffe zur Verbesserung der Haarstruktur haben sich kationische Tenside, insbesondere quaternäre Ammoniumverbindungen wie beispielsweise Distearyldimethylammoniumchlorid (DSDMAC) alleine oder in Kombination mit verschiedenen wachsartigen Zusätzen, wie Kohlenwasserstoffen, Fettalkoholen oder Fettsäureestern bewährt [**Parf.Kosm. 56, 157 (1975)**].

Von Nachteil ist hierbei jedoch, daß die genannten Kationtenside eine unzureichende biologische Abbaubarkeit aufweisen und somit bei Eintragung in Oberflächengewässer im Laufe der Zeit die Funktionsfähigkeit aquatischer Lebensgemeinschaften beeinträchtigen können.

Gemäß der Lehre der **EP-A 0309052** ist die Verwendung von quaternierten Difettsäurealkanolaminestern als textile Weichmacher und Haarkonditioniermittel bekannt. Die **EP-A 0284036** offenbart ebenfalls den Einsatz von kationischen Tensiden mit Esterstruktur für haarkosmetische Anwendungen. Die **EP-A 0252441** betrifft Veresterungsprodukte von Etheraminen, die anschließend quaterniert werden. Die **EP-A 0299787** ist daneben ausschließlich auf den Einsatz von Esterquats als textile Wäscheweichspülmittel gerichtet.

Aus der Deutschen Patentanmeldung **DE-A1 3527974** sind darüber hinaus Ester des Betains mit Fettalkoholen oder Fettalkoholpolyglycolethern für den Einsatz in sauren Haarpflegemitteln bekannt. Die Betainester weisen zwar eine hohe ökotoxikologische Verträglichkeit auf, sind jedoch im Hinblick auf Kämmbarkeitsverbesserung, Antistatik, Griff und Ausspülverhalten unbefriedigend und zudem im sauren Bereich nicht hydrolysestabil.

Die Aufgabe der Erfindung bestand somit darin, neue Haarpflegemittel zu entwickeln, die frei von den geschilderten Nachteilen sind.

### Beschreibung der Erfindung

### Gegenstand der Erfindung ist die Verwendung von Gemischen, enthaltend

(a) quaternierte FettsäuretrialkanolaminesterSalze der Formel **(I)** 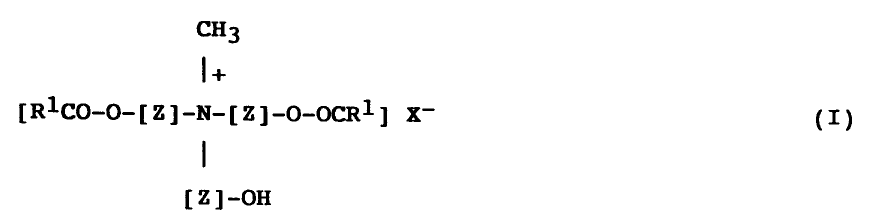 in der R¹CO für einen linearen oder verzweigten, aliphatischen Acylrest mit 6 bis 22 Kohlenstoffato men und 0 oder 1 Doppelbindung, [Z] für eine Ethylen-, Propylen- oder Isopropylen-Gruppe und X für Chlorid, Bromid, Sulfat, Methosulfat oder Phosphat steht, und
(b) Fettalkohole und/oder Fettalkoholpolyglycolether zur Herstellung von Haarspülungen, Haarpflegeemulsionen, Haarkuren, Aerosolschäumen und Fönlotionen.

Überraschenderweise wurde gefunden, daß die erfindungsgemäßen Gemische die Trocken- und Naßkämmbarkeit von Haaren auf bis zu 20 % des Ausgangswertes vermindern können und eine elektrostatische Aufladung beim Trockenkämmen nahezu vollständig unterdrücken. Die Produkte verleihen dem Haar einen weichen Griff und lassen sich leicht wieder ausspülen. Im Gegensatz zu den gängigen Produkten des Marktes sind sie sowohl aerob als auch anaerob vollständig biologisch abbaubar und zeichnen sich durch eine unerwartet geringe akute bzw. chronische Toxizität gegenüber aquatischen Lebensgemeinschaften aus. Des weiteren sind sie sowohl im alkalischen als auch sauren pH-Bereich außerordentlich hydrolyse- und lagerstabil.

### Quaternierte Fettsäuretrialkanolaminester-Salze

Quaternierte Fettsäuretrialkanolaminester-Salze stellen bekannte Stoffe dar, die nach den einschlägigen Methoden der präparativen organischen Chemie erhalten werden können. Zu ihrer Herstellung geht man beispielsweise von Triethanolamin aus, das mit Fettsäuren verestert und anschließend mit Dimethylsulfat quaterniert wird. Die Verwendung derartiger Stoffe als Avivagemittel für Textilien ist beispielsweise aus der Europäischen Patentanmeldung **EP-A2 0370675** bekannt.

Typische Beispiele für quaternierte Fettsäuretrialkanolaminester-Salze sind Difettsäureester von Triethanolamin, Tripropanolamin und Tri-i-propanolamin mit Capronsäure, Caprylsäure, Caprinsäure, Laurinsäure, Myristinsäure, Palmitinsäure, Stearinsäure, Ölsäure, Elaidinsäure, Petroselinsäure, Arachinsäure, Behensäure und Erucasäure, die mit Methylchlorid, Dimethylsulfat oder Dimethylphosphat quaterniert worden sind. Mittel mit besonders vorteilhaften anwendungstechnischen Eigenschaften weisen einen Gehalt von quaternierten Fettsäuretrialkanolaminester-Salzen der Formel **(I)** auf, in der R¹CO für einen Acylrest mit 16 bis 18 Kohlenstoffatomen, [Z] für eine Ethylengruppe und X für Methosulfat steht.

Wie in der Fettchemie üblich, können zur Herstellung der quaternierten Fettsäuretrialkanolaminester-Salze auch technische Fettsäureschnitte eingesetzt werden, wie sie bei der Druckspaltung von Fetten und Ölen, beispielsweise Palmöl, Palmkernöl, Kokosöl oder Rindertalg anfallen. Bevorzugt sind quaternierte Estersalze, deren Fettsäurekomponente sich von Fettsäuren mit 12 bis 18, vorzugsweise 16 bis 18 Kohlenstoffatomen ableitet. Als besonders vorteilhaft haben sich darüberhinaus quaternierte Estersalze erwiesen, die auf Basis von technischer Elaidinsäure, d. h. einer Octadecen-9-säure mit einem Gehalt an trans-ständigen Doppelbindungen von 35 bis 95, vorzugsweise 40 bis 70 Gew.-%, hergestellt werden.

Die Mittel können die Estersalze der Formel **(I)** in Mengen von 0,1 bis 25, vorzugsweise 1 bis 10 Gew.-% - bezogen auf die Mittel - enthalten.

Der pH-Wert der sauren Haarpflegemittel kann im Bereich von 2 bis 5, vorzugsweise 2,5 bis 4,5 liegen. Er kann beispielsweise mit Hilfe von Essigsäure, Milchsäure, Citronensäure, Salzsäure, Phosphorsäure oder Betainhydrochlorid eingestellt werden.

Obschon die Mittel auch als saure Lösungen oder Suspensionen der quaternierten Fettsäuretrialkanolaminester-Salze in Wasser auf den Markt gebracht werden können, enthalten sie bevorzugt weitere, in kosmetischen Mitteln übliche Bestandteile, wie beispielsweise Fettalkohole, Fettalkoholpolyglycolether, Fettsäureester, Konservierungsmittel, Vitamine und Wachse.

Die folgenden Beispiele sollen den Gegenstand der Erfindung näher erläutern, ohne ihn darauf einzuschränken.

### Beispiele

### I. Eingesetzte quaternierte Fettsäuretrialkanolaminester-Salze

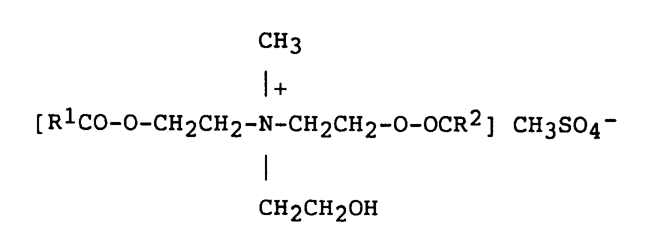

- A1) R¹CO =: C_{16/18}-Acylrest,
Basis Talgfettsäure
- A2) R¹CO =: C_{16/18}-Acylrest,
Basis Palmfettsäure
- A3) R¹CO =: C_{16/18}-Acylrest,
Basis technische Elaidinsäure:
40 Gew.-% trans-Octadecen-9-säure
60 Gew.-% cis-Octadecen-9-säure

### II. Eingesetzte Rezepturen

**Tab.1**

| Haarpflegemittel-Rezepturen | | | | | |
|---|---|---|---|---|---|
| Komponenten Gew.-% | Rezeptur | | | | |
| | A | B | C | D | E |
| A1 | - | - | 1,2 | - | - |
| A2 | - | - | - | 1,2 | - |
| A3 | - | - | - | - | 1,2 |
| Dehyquart DAM | - | 1,3 | - | - | - |
| Emulgade 1000 NI | 4,0 | 4,0 | 4,0 | 4,0 | 4,0 |
| Kathon CG | 0,07 | 0,07 | 0,07 | 0,07 | 0,07 |
| Wasser | ad 100 | | | | |
| pH-Wert | 4,0 | | | | |

### Legende:

- Dehyquart^{(R)} DAM =: Distearyldimethylammoniumchlorid
- Emulgade ^{(R)} 1000 NI =: C_{16/18}-Fettalkohol/ C_{16/18}-Fettalkohol-20EO-Addukt (Gewichtsverhältnis 1 : 1)
- Kathon^{(R)} CG =: Konservierungsmittel
Die Rezepturen C, D und E sind erfindungsgemäß, die Rezepturen A und B dienen dem Vergleich.

### III. Anwendungstechnische Beispiele

a) Trockenkämmbarkeit/Elektrostatische Aufladung
   Die elektrostatische Aufladung wurde parallel mit der Trockenkämmbarkeit unter Zulassung der elektrostatischen Aufladung untersucht. Es wurde eine relative Luftfeuchtigkeit von 20 % eingestellt. Die Konditionierungszeit betrug 12 h bei 30°C. Die Messung erfolgte über den Ladungsabgriff an einem doppelten Faraday-Käfig nach Ausführung von 10 Kämmungen. Der Fehler bei den Messungen betrug im Mittel 2,5 %, die statistische Sicherheit lag bei mindestens 99,9 %. Die Ergebnisse der Kämmarbeiten sind in Tab.2 und 3 zusammengefaßt.
b) Naßkämmbarkeit
   Die Naßkämmbarkeit wurde an braunem Haar (Alkinco #6634, Strähnenlänge 12 cm, Strähnenmasse 1 g) untersucht. Nach der Nullmessung wurden die Strähnen mit 100 ml der Formulierungen A bis E getränkt. Nach einer Einwirkzeit von 5 min wurden die Strähnen 1 min unter fließendem Wasser (1 l/min, 38°C) ausgespült. Die Strähnen wurden erneut vermessen und mit der Nullmessung verglichen. Der Fehler bei den Messungen betrug im Mittel 2 %, die statistische Sicherheit lag bei mindestens 99 %. Die Ergebnisse sind in Tab.4 zusammengefaßt.

Eine ausführliche Beschreibung der Meßmethoden befindet sich in **J.Soc.Cosm.Chem., 24, 782 (1973.**

**Tab.2**

| Trockenkämmbarkeit | | | |
|---|---|---|---|
| Bsp. | Rezeptur | Trockenkämmbarkeit [mJ] | |
| | | vorher | nachher |
| 1 | C | 4,5 | 0,8 |
| 2 | D | 4,0 | 0,7 |
| 3 | E | 4,1 | 1,2 |
| V1 | A | 5,3 | 3,1 |
| V2 | B | 3,6 | 0,8 |

**Tab.3**

| Elektrostatische Aufladung | | | |
|---|---|---|---|
| Bsp. | Rezeptur | Elstat. Aufladung [V] | |
| | | vorher | nachher |
| 4 | C | 1,8 | -0,2 |
| 5 | D | 2,1 | -0,2 |
| 6 | E | 2,2 | -0,1 |
| V2 | A | 2,5 | 1,2 |
| V3 | B | 2,2 | -0,2 |

**Tab.4**

| Naßkämmbarkeit | | | |
|---|---|---|---|
| Bsp. | Rezeptur | Naßkämmbarkeit [mJ] | |
| | | vorher | nachher |
| 7 | C | 24,0 | 7,3 |
| 8 | D | 23,6 | 4,5 |
| 9 | E | 20,9 | 4,9 |
| V5 | A | 27,4 | 18,5 |
| V6 | B | 23,7 | 3,8 |

## Patentansprüche

1. Verwendung von Gemischen, enthaltend
(a) quaternierte FettsäuretrialkanolaminesterSalze der Formel **(I)** 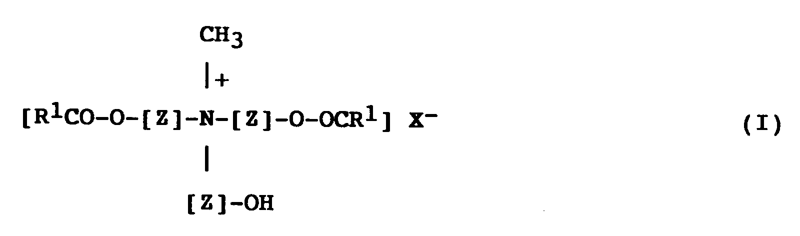 in der R¹CO für einen linearen oder verzweigten, aliphatischen Acylrest mit 6 bis 22 Kohlenstoffatomen und 0 oder 1 Doppelbindung, [Z] für eine Ethylen-, Propylen- oder Isopropylen-Gruppe und X für Chlorid, Bromid, Sulfat, Methosulfat oder Phosphat steht, und
(b) Fettalkohole und/oder Fettalkoholpolyglycolether
zur Herstellung von Haarspülungen, Haarpflegeemulsionen, Haarkuren, Aerosolschäumen und Fönlotionen.

2. Verwendung nach Anspruch 1, **dadurch gekennzeichnet**, daß R¹CO für einen Acylrest mit 16 bis 18 Kohlenstoffatomen steht.

3. Verwendung nach den Ansprüchen 1 und 2, **dadurch gekennzeichnet**, daß [Z] für eine Ethylengruppe steht.

4. Verwendung nach den Ansprüchen 1 bis 3, **dadurch gekennzeichnet**, daß X für Methosulfat steht.

5. Verwendung nach den Ansprüchen 1 bis 4, **dadurch gekennzeichnet**, daß Estersalze der Formel (I) in Mengen von 0,1 bis 25 Gew.-% - bezogen auf die Mittel - enthalten sind.

6. Verwendung nach den Ansprüchen 1 bis 5, **dadurch gekennzeichnet**, daß der pH-Wert 2 bis 5 beträgt.

## Claims

1. The use of mixtures containing
(a) quaternized fatty acid trialkanolamine ester salts corresponding to formula (I): 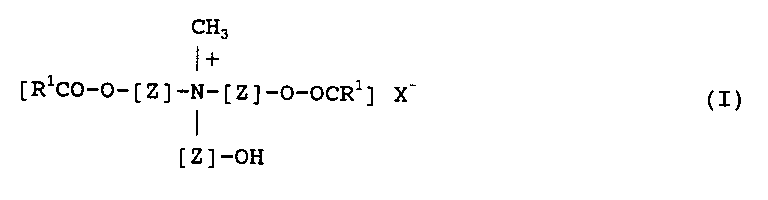 in which
R¹CO is a linear or branched aliphatic acyl radical containing 6 to 22 carbon atoms and 0 or 1 double bond, [Z] is an ethylene, propylene or isopropylene group and X is chloride, bromide, sulfate, methosulfate or phosphate, and
(b) fatty alcohols and/or fatty alcohol polyglycol ethers
for the production of hair rinses, hair-care emulsions, hair tonics, aerosol foams and setting lotions.

2. The use claimed in claim 1, characterized in that R¹CO is an acyl radical containing 16 to 18 carbon atoms.

3. The use claimed in claims 1 and 2, characterized in that [Z] is an ethylene group.

4. The use claimed in claims 1 to 3, characterized in that X represents methosulfate.

5. The use claimed in claims 1 to 4, characterized in that ester salts corresponding to formula (I) are present in quantities of 0.1 to 25% by weight, based on the preparation.

6. The use claimed in claims 1 to 5, characterized in that the pH value is in the range from 2 to 5.

## Revendications

1. Utilisation de mélanges renfermant :
a) des sels d'esters de trialcanolamines d'acides gras quaternisés de formule I, 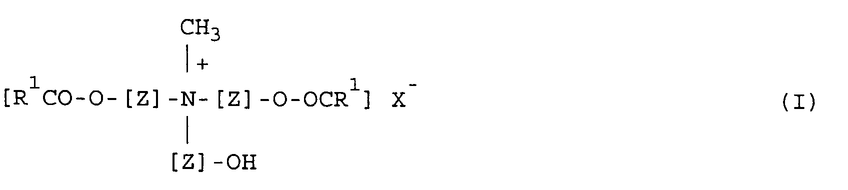 dans laquelle R¹CO représente un radical acyle aliphatique, linéaire ou ramifié, ayant de 6 à 22 atomes de carbone et 0 ou une double liaison,
Z représente un groupe éthylène, propylène ou isopropylène et
X représente un chlorure, un bromure, un sulfate, un méthosulfate ou un phosphate et
b) des alcools gras et/ou des éthers d'alcool gras de polyglycols,
pour la production de rinçages pour les cheveux, d'émulsions pour le soin des cheveux, de traitements pour les cheveux, de mousses en aérosol, et de lotions pour sèchecheveux.

2. Utilisation selon la revendication 1, caractérisée en ce que R¹CO représente un radical acyle ayant de 16 à 18 atomes de carbone.

3. Utilisation selon les revendications 1 et 2, caractérisée en ce que [Z] représente un groupe éthylène.

4. Utilisation selon les revendications 1 à 3, caractérisée en ce que X représente un méthosulfate.

5. Utilisation selon les revendications 1 à 4, caracterisée en ce que les sels d'ester de formule (I) sont inclus en quantités allant de 0,1 à 25 % en poids, rapporté à l'agent.

6. Utilisation selon les revendications 1 à 5, caractérisée en ce que la valeur du pH s'élève de 2 à 5.
